# EUROPEAN PATENT APPLICATION

(11) **EP 2 907 877 A1**
(43) Date of publication of application: **19.08.2015**
(21) Application number: 13844953.3
(22) Date of filing: 10.10.2013
(51) Int. Cl.: C12Q 1/02, A61K 49/00, C12Q 1/48

(54) **REAGENT FOR IMAGING INTRACELLULAR ACETYLATION**

(30) Priority: 11.10.2012 JP 2012225841
(71) Applicant: Japan Science and Technology Agency, Kawaguchi-shi, Saitama 332-0012 (JP)
(72) Inventor: KANAI, Motomu, Tokyo 112-0015 (JP); KOMATSU, Hirokazu, Tsukuba-shi Ibaraki 305-0051 (JP)
(74) Representative: Godemeyer Blum Lenze Patentanwälte Partnerschaft mbB - werkpatent
(86) International application number: PCT/JP2013/077542
(87) International publication number: WO 2014/057999

(57) **Abstract**

A reagent for imaging an advancing intracellular acetylation, for example, an acetylation that is advanced by the action of acetyl-CoA in the mitochondria, at high sensitivity and high efficiency, which comprises a combination of a rhodamine derivative that is substantially non-fluorescent before being acetylated, and emits strong fluorescence after being acetylated, and an acylation catalyst and/or an acylation reaction-promoting agent.

## Description

### Technical Field

The present invention relates to a reagent that is capable of highly accurate and convenient imaging of an intracellularly advancing acetylation.

### Background Art

Variety of fluorescent probes have hitherto been proposed and practically used that react with measurement objects including ion species such as calcium ion, nitric oxide, reactive oxygen species and the like existing in living bodies and thereby enable imaging of the measurement objects (for example, Fluo-4 and the like are widely used as a fluorescent probe for detection of calcium ion). These fluorescent probes are characterized in that they are substantially non-fluorescent or weakly fluorescent in the absence of a measurement object, whilst specifically capture a measurement object or specifically react with a measurement object to emit strong fluorescence. There have also been proposed fluorescent probes that react with a peptidase existing in living bodies and are hydrolyzed to emit strong fluorescence. The probes are also applied to diagnosis of cancers (International Patent Publication WO2011/87000).

As the fluorophore for constituting such fluorescent probes, mother nucleus structures such as fluorescein structure, rhodamine structure and indacene structure have been used (see, for example, Japanese Patent Unexamined Publication (KOKAI) Nos. 10-338695, 11-5796 and the like). Rhodamine is a fluorescent dye known for many years like fluorescein, and since it shows a high fluorescence quantum yield in water, it is widely used in the field of biology as a fluorescent labeling agent. As fluorescent probes having the rhodamine structure, there have been proposed, for example, the fluorescent probe for detection of nitric oxide (International Patent Publication WO1999/001447), fluorescent probe for detection of hypochlorous acid (International Patent Publication WO2007/100061), and the like.

Typical examples of the important intracellular acetylation include the acetylation of histone. In this acetylation, acetyl-CoA produced via the tricarboxylic acid cycle in the mitochondria is used as a catalytic acylating agent. Mitochondria are intracellular organelles that produce ATP as an energy source for the cellular metabolism, and it is known that, since they have an electron transport system, they can be stained with various oxidation/reduction dyes. In particular, it is known that a positively charged low molecular weight organic dye can quickly penetrate the cell membrane and stain the mitochondrial membrane, and the mitochondrial membrane potential dependency of the degree of staining with such a dye is used for evaluation of intracellular functions of mitochondria. Examples of such a positively charged organic dye include rhodamine 123. Rhodamine 123 easily penetrates the cell membrane, and is accumulated in the mitochondria to give absorption and excitation/emission spectral changes depending on the membrane potential of the mitochondria (reflecting ATP generation ability). Therefore, it is supposed that ATP production amount in the mitochondria can be estimated by using rhodamine 123 (Biochim. Biophys. Acta, 850, 436-448, 1986).

However, no techniques have so far been developed for imaging an acetylation that advances intracellularly, especially the acetylation using acetyl-CoA produced in the mitochondria which participate in the energy production, at high sensitivity and high efficiency, and development of such techniques has strongly been desired. In addition, imaging of various kinds of reactions advancing in a living body or cell, such as the intracellular acetylation, by using a fluorescent probe and an artificial catalyst or reaction accelerator in combination in a living body or cell.

### Prior art references

### Non-patent documents

Non-patent document 1: International Patent Publication WO1999/001447
Non-patent document 2: International Patent Publication WO2007/100061
Non-patent document 3: Biochim. Biophys. Acta, 850, 436-448, 1986

### Summary of the Invention

### Object to be Achieved by the Invention

An object of the present invention is to provide a means for imaging an acetylation advancing intracellularly cell, for example, the acetylation that is advanced by the action of acetyl-CoA in the mitochondria, at high sensitivity and high efficiency.

### Means for Achieving the Object

The inventors of the present invention conducted various researches in order to achieve the aforementioned object. As a result, they found that, by using a rhodamine derivative that is substantially non-fluorescent before being acetylated, and emits strong fluorescence after being acetylated as a fluorescent probe in combination with an acylation catalyst or acylation reaction-promoting agent such as tributylphosphine, intracellular acetylation was successfully and highly sensitively measured in a short time, and for example, the intracellular acetylation that is advanced by the action of acetyl-CoA in the mitochondria was successfully and conveniently imaged. The present invention was accomplished on the basis of the aforementioned finding.

The present invention thus provides a reagent for visualizing an intracellular acetylation, which comprises a combination of a rhodamine derivative that is substantially non-fluorescent before being acetylated, and emits strong fluorescence after being acetylated, and an acylation catalyst and/or an acylation reaction-promoting agent.

According to preferred embodiments of the present invention, there are provided the aforementioned reagent, wherein the intracellular acetylation is an acetylation that is advanced by acetyl-CoA; and the aforementioned reagent, which is for visualizing an acetylation with acetyl-CoAproduced by an intracellular mitochondrion.

As other aspects of the present invention, there are provided a reagent for measuring intracellular acetyl-CoA, which comprises a combination of a rhodamine derivative that is substantially non-fluorescent before being acetylated, and emits strong fluorescence after being acetylated, and an acylation catalyst and/or an acylation reaction-promoting agent; and a reagent for visualizing intracellular acetyl-CoA, which comprises a combination of a rhodamine derivative that is substantially non-fluorescent before being acetylated, and emits strong fluorescence after being acetylated, and an acylation catalyst and/or an acylation reaction-promoting agent.

As further aspects of the present invention, there are provided a kit for visualizing an intracellular acylation reaction, which comprises a combination of a rhodamine derivative that is substantially non-fluorescent before being acetylated, and emits strong fluorescence after being acetylated, and an acylation catalyst and/or an acylation reaction-promoting agent; and a kit for measuring intracellular acetyl-CoA, which comprises a combination of a rhodamine derivative that is substantially non-fluorescent before being acetylated, and emits strong fluorescence after being acetylated, and an acylation catalyst and/or an acylation reaction-promoting agent.

The present invention also provides a rhodamine derivative to be used in combination with an acylation catalyst and/or an acylation reaction-promoting agent for visualizing an intracellular acetylation, which is substantially non-fluorescent before being acetylated, and emits strong fluorescence after being acetylated; and an acylation catalyst and/or an acylation reaction-promoting agent to be used in combination with a rhodamine derivative that is substantially non-fluorescent before being acetylated, and emits strong fluorescence after being acetylated, for visualizing an intracellular acetylation.

As still further aspects, the present invention provides a method for visualizing an intracellular acetylation, which comprises the step of introducing a combination of a rhodamine derivative that is substantially non-fluorescent before being acetylated, and emits strong fluorescence after being acetylated, and an acylation catalyst and/or an acylation reaction-promoting agent into a cell, and the step of measuring fluorescence of the rhodamine derivative having been acetylated; and a method for measuring intracellular acetyl-CoA, which comprises the step of introducing a combination of a rhodamine derivative that is substantially non-fluorescent before being acetylated, and emits strong fluorescence after being acetylated, and an acylation catalyst and/or an acylation reaction-promoting agent into a cell, and the step of measuring fluorescence of the rhodamine derivative having been acetylated.

### Effect of the Invention

In living bodies, acetylations occur with various substances as objects that exist in the living bodies, which include, for example, acetylation of proteins after translation, and acetylation of histone that controls gene expression. These acetylations occur as enzyme-catalyzed reactions involving acetyl-CoA. Acetyl-CoA existing in living bodies is produced in mitochondria, and participates in the TCA cycle. Therefore, visualization of an acetylation in a living body allows imaging of acetyl-CoA. Imaging acetyl-CoA to confirm its distribution or localization enables, for example, pathological and physiological elucidation of cause of onset or advance of pathological conditions of diseases such as arteriosclerosis and hyperlipidemia at a cellular level.

### Brief Description of the Drawings

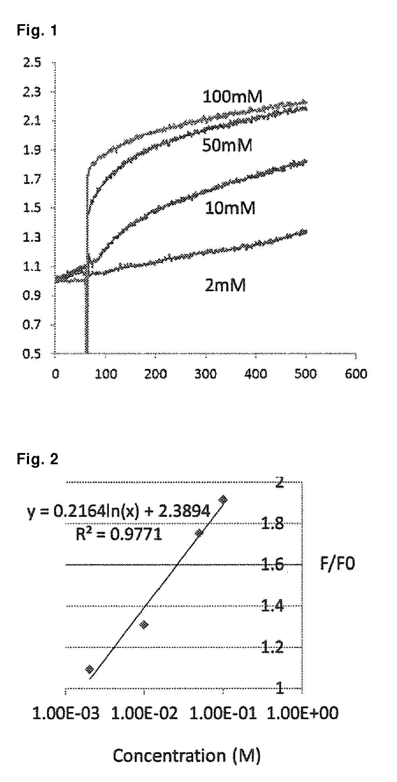

[Fig. 1] Fig. 1 shows the results of measurement of advance of acetylation over time, in which the compound 3 (1 µM) as a rhodamine derivative and tributylphosphine (10 mM) as an acylation catalyst were mixed, the compound 6 as an acetylating agent was added 1 minute afterward, and advance of acetylation was measured over time.
[Fig. 2] Fig. 2 shows correlation of concentration of the compound 6 and increase in fluorescence intensity 1 minute after the addition of the compound 6.

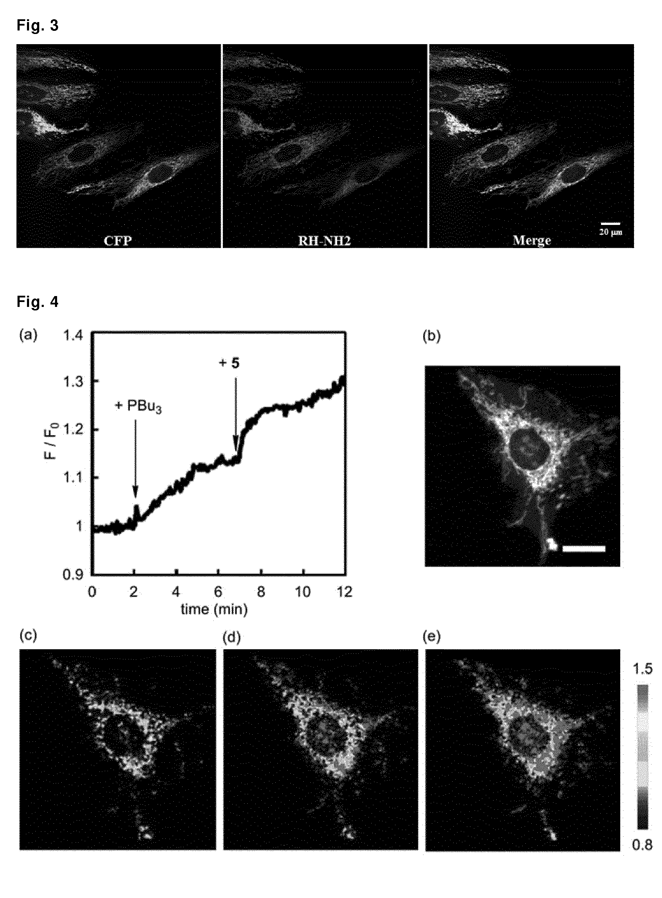

[Fig. 3] Fig. 3 shows the results of double staining of the HeLa cells with the compound 3 as a rhodamine derivative and cyan fluorescence protein (CFP). The left photograph shows the result obtained with CFP alone (excitation, 440 nm; fluorescence, 460 to 500 nm), the central photograph shows the result obtained with the compound 3 (RH-NH₂) alone (excitation, 559 nm; fluorescence, 570 to 670 nm), and the right photograph shows the overlapped state of the foregoing photographs.
[Fig. 4] Fig. 4 shows the results of visualization of acetylation attained with the compound 3 (10 µM) in the HeLa cells. Fig. 4, (a) shows enhancement of fluorescence over time attained by acetylation induced at 37°C in the HeLa cell with tributylphosphine (5 mM) as an acylation catalyst. The acylation catalyst was added at the time point of 2 minutes, and N-methoxydiacetamide (NMD, 10 mM) was added as an acetylating agent at the time point of 7 minutes. Enhancement of the fluorescence intensity (F/F₀) was calculated with an excitation wavelength of 550 nm and a fluorescence wavelength of 575 nm as an average of the results obtained from five cells. Fig. 4, (b) shows a fluorescence image of the HeLa cell, wherein the scale bar indicates 10 µm. Figs. 4, (c), (d), and (e) show color images of the HeLa cell after 1 minute, 5 minutes, and 10 minutes, respectively.

### Modes for Carrying out the Invention

The rhodamine derivative used in the present invention may be those substantially non-fluorescent before being acetylated, whilst strongly fluorescent after being acetylated, and the chemical structure thereof is not particularly limited. In this specification, the term rhodamine derivative means a compound comprising xanthene having two amino groups at the 3- and 6-positions (one of these is quaternary amino group) and phenyl group at the 9-position as the basic skeleton, or such a compound in which the oxygen atom of the xanthene is replaced with a dialkyl-substituted silicon atom, germanium atom, or tin atom, and the scope thereof will be easily understood by those skilled in the art. As for the property that the rhodamine derivative is substantially non-fluorescent before being acetylated, while emits strong fluorescence after being acetylated, it can be readily determined whether a rhodamine derivative is suitable for the purpose of the present invention by measuring the fluorescence spectra of the derivative before and after acetylation of the amino group on which R¹ substitutes.

Preferred examples of the rhodamine derivatives usable in the present invention include, for example, a rhodamine derivative represented by the following general formula (I): 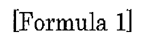 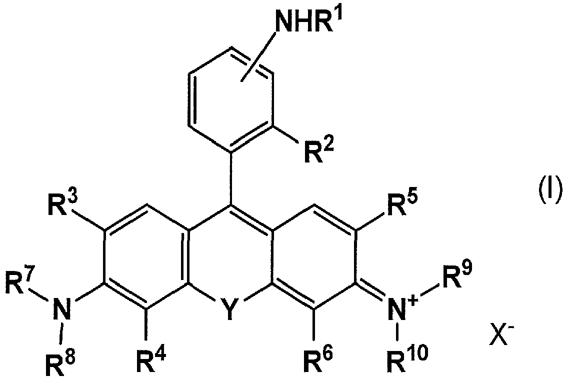 wherein, in the formula, R¹ represents hydrogen atom or a C₁₋₆ alkyl group; R² represents hydrogen atom, a C₁₋₆ alkyl group, or carboxyl group; R³ and R⁵ independently represent hydrogen atom, a halogen atom, or a C₁₋₆ alkyl group; R⁴ and R⁶ independently represent hydrogen atom or a C₁₋₆ alkyl group; R⁷ and R⁸ independently represent a C₁₋₆ alkyl group, but the alkyl group represented by R⁷ and the alkyl group represented by R³ may bind together to form a 5- to 7-membered ring, and/or the alkyl group represented by R⁸ and the alkyl group represented by R⁴ may bind together to form a 5- to 7-membered ring; R⁹ and R¹⁰ independently represent a C₁₋₆ alkyl group, but the alkyl group represented by R⁹ and the alkyl group represented by R⁵ may bind together to form a 5- to 7-membered ring, and/or the alkyl group represented by R¹⁰ and the alkyl group represented by R⁶ may bind together to form a 5- to 7-membered ring; X- represents a counter ion; and Y represents oxygen atom or M(R¹¹)(R¹²) (M represents silicon atom, germanium atom, or tin atom; and R¹¹ and R¹² independently represents a C₁₋₆ alkyl group), but the rhodamine derivatives usable in the present invention are not limited to the aforementioned specific rhodamine derivative.

In the aforementioned general formula (I), the term alkyl group encompasses linear, branched and cyclic alkyl groups, and alkyl groups consisting of a combination of these. The halogen atom may be any of fluorine atom, chlorine atom, bromine atom, and iodine atom.

The position on the benzene ring at which -NHR¹ binds is not particularly limited, and -NHR¹ may binds at an arbitrary substitutable position on the benzene ring. However, -NHR¹ preferably substitutes at the meta-position or para-position with respect to R², particularly preferably at the meta-position with respect to R². Although it is preferred that R¹ is hydrogen atom, it may also be preferred that R¹ is methyl group or ethyl group.

R² represents hydrogen atom, a C₁₋₆ alkyl group, or carboxyl group, and the carboxyl group may be in the form of ester thereof. For example, R² may be a C₁₋₈ alkoxycarbonyl group, a C₁₋₈ alkoxyalkoxycarbonyl group, or the like. R² preferably represents carboxyl group. In this case, the counter ion represented by X may not exist, and there may be an intramolecular counter ion. It is preferred that R² is hydrogen atom or carboxyl group, and it is more preferred that R² is hydrogen atom.

R³ and R⁵ independently represent hydrogen atom, a halogen atom, or a C₁₋₆ alkyl group. As the halogen atom, fluorine atom or chlorine atom is preferred. It is preferred that R³ and R⁵ are hydrogen atoms. R⁴ and R⁶ independently represent hydrogen atom or a C₁₋₆ alkyl group, and it is preferred that R⁴ and R⁶ are hydrogen atoms.

R⁷ and R⁸ independently represent a C₁₋₆ alkyl group, and it is preferred that both R⁷ and R⁸ are methyl groups or ethyl groups. The alkyl group represented by R⁷ and the alkyl group represented by R³ may bind together to form a 5- to 7-membered ring. The alkyl group represented by R⁸ and the alkyl group represented by R⁴ may bind together to form a 5- to 7-membered ring. R⁹ and R¹⁰ independently represent a C₁₋₆ alkyl group, and it is preferred that both R⁹ and R¹⁰ are methyl groups or ethyl groups. The alkyl group represented by R⁹ and the alkyl group represented by R⁵ may bind together to form a 5- to 7-membered ring. The alkyl group represented by R¹⁰ and the alkyl group represented by R⁶ may bind together to form a 5- to 7-membered ring.

X⁻ represents a counter ion. Type of the counter ion is not particularly limited, and an arbitrary counter ion having electrical charge matching the positive charge of the nitrogen atom can be used. For example, a halogen ion such as chlorine ion, bromine ion, and iodine ion, as well as an organic acid ion, and the like may be used. When R² is carboxyl group, the counter ion may not exist. Y represents oxygen atom or M(R¹¹)(R¹²). M represents silicon atom, germanium atom, or tin atom, and it is preferred that M is silicon atom. R¹¹ and R¹² independently represent a C₁₋₆ alkyl group, and it is preferred that both R¹¹ and R¹² are methyl groups, or the like.

Preferred embodiments of the rhodamine derivative represented by the general formula (I) include the rhodamine derivative in which R¹ is hydrogen atom or a C₁₋₆ alkyl group; R² is hydrogen atom, a C₁₋₆ alkyl group, or carboxyl group; R³ and R⁵ are hydrogen atoms; R⁴ and R⁶ are hydrogen atoms; R⁷ and R⁸ are independently C₁₋₆ alkyl groups; R⁹ and R¹⁰ are independently C₁₋₆ alkyl groups; X⁻ is a counter ion; and Y is oxygen atom, and further preferred embodiments of the rhodamine derivative include the rhodamine derivative in which R¹ is hydrogen atom; R² is hydrogen atom; R³ and R⁵ are hydrogen atoms; R⁴ and R⁶ are hydrogen atoms; R⁷ and R⁸ are independently C₁₋₆ alkyl groups; R⁹ and R¹⁰ are independently C₁₋₆ alkyl groups; X⁻ is a counter ion; and Y is oxygen atom.

Particularly preferred rhodamine derivatives include the rhodamine derivative in which R¹ is hydrogen atom; R² is hydrogen atom; R³, R⁴, R⁵, and R⁶ are hydrogen atoms; R⁷, R⁸, R⁹, and R¹⁰ are methyl groups; X⁻ is a counter ion; and Y is oxygen atom, and as an example thereof, N-(9-(4-aminophenyl)-6-(dimethylamino)-3H-xanthen-3-ylidene)-N-methylmethanaminium salt (compound 3) described in the examples can be mentioned. However, the rhodamine derivatives usable in the present invention are not limited to the aforementioned specific rhodamine derivatives.

Since various rhodamine derivatives have been reported, those skilled in the art can easily prepare the rhodamine derivative used for the present invention by a known method with appropriately choosing the starting materials and reagents. The rhodamine derivatives in which Y is Si(R¹¹)(R¹²), and use thereof as a fluorescent probe are reported in Best, Q et al., Pacifichem., 2010, Subject number 2335, December 19, 2010; Yuichiro Koide et al., 4th Convention of Japanese Society for Molecular Imaging, Subject number P8-9, May 14, 2009 and the like, the preparation methods and use as a fluorescent probe of the rhodamine derivatives in which Y is M(R¹¹)(R¹²) (M is silicon atom, germanium atom, or tin atom) are described in International Patent Publication WO2012/111818, and therefore those skilled in the art can also easily prepare the rhodamine derivatives in which Y is M(R¹¹)(R¹²) (M is silicon atom, germanium atom, or tin atom) by referring to those publications. The entire disclosures of International Patent Publication WO2012/111818 mentioned above are incorporated into the disclosure of this specification by reference.

As the acylation catalyst or acylation reaction-promoting agent, an arbitrary acylation catalyst or acylation reaction-promoting agent can generally be used, so long that an agent is chosen which can catalyze or promote a nucleophilic acyl substitution reaction that gives an acyl compound by a reaction of an acyl compound such as ester, amide, carboxylic acid halide, or carboxylic anhydride with a nucleophilic agent. Type of the acylation catalyst or acylation reaction-promoting agent is not particularly limited. When acetylation with intracellular acetyl-CoA is measured by using the reagent of the present invention, acetyl-CoA as an acyl compound reacts with the amino group on the benzene ring of the rhodamine derivative that acts as a nucleophilic agent, so that the amino group is acetylated, and an acylation catalyst or acylation reaction-promoting agent that can efficiently catalyze or promote this reaction can be preferably used. As the acylation catalyst or acylation reaction-promoting agent, an acylation catalyst or acylation reaction-promoting agent consisting of a low molecular weight organic compound can generally be used. Various kinds of acylation catalysts or acylation reaction-promoting agents known as asymmetric acylation catalyst or acylation reaction-promoting agent can also be used (for example, Vedejs E. SYNLETT, 10, 1499-1505, 2001, all the references cited in this reference and the like). The acylation catalyst and the acylation reaction-promoting agent can be used in an appropriate combination. Embodiments using an acylation catalyst having an acylation reaction-promoting action, or an acylation reaction-promoting agent having an acylation catalyzing action also fall within the scope of the present invention. In such a case, an acylation catalyst having an acylation reaction-promoting action may be referred to simply as "acylation catalyst", and an acylation reaction-promoting agent having an acylation catalyzing action may be referred to simply as "acylation reaction-promoting agent".

For example, there can be preferably used, but not limited to, dialkylaminopyridines such as dimethylaminopyridine (DMAP), cyclic tertiary organic amines such as 1,4-diazabicyclo[2.2.2]octane (DABCO) and 1,8-diazabicyclo[5.4.0]undec-7-ene (DBU), phosphines including trialkylphosphines such as tributylphosphine, and triarylphosphines such as triphenylphosphine, and the like, which are widely used for acetylations. The acylation catalyst or acylation reaction-promoting agent should be appropriately chosen from the viewpoints of the catalytic activity, reaction promotion activity for the rhodamine derivative and acyl compound to be used, intracellular migration property, and cytotoxicity, as well as intramitochondrial migration property and the like in the case of, for example, measurement of acetylation with acetyl-CoA in mitochondria.

Ratio of the rhodamine derivative and the acylation catalyst or acylation reaction-promoting agent in the reagent of the present invention is not particularly limited, and can be appropriately chosen depending on type of the acetylation as the object of the visualization, existing site of the acetylating agent, types of the rhodamine derivative, acylation catalyst or acylation reaction-promoting agent to be used and the like. For example, the acylation catalyst and/or acylation reaction-promoting agent can be used in an amount of about 100 to 1,000,000 times of the amount of the rhodamine derivative. For example, when acetyl-CoA is measured in mitochondria by using N-(9-(4-aminophenyl)-6-(dimethylamino)-3H-xanthen-3-ylidene)-N-methylmethanaminium salt as the rhodamine derivative, and a trialkylphosphine compound such as tributylphosphine as the acylation catalyst or acylation reaction-promoting agent, the acylation catalyst or acylation reaction-promoting agent can be used at a concentration of about 1,000 to 100,000 times, preferably about 5,000 to 50,000 times, particularly preferably about 10,000 times, of the concentration of the rhodamine derivative. However, the concentration is not limited to the aforementioned specific ranges.

The reagent of the present invention can be used for visualizing an intracellular acetylation. Although type of the intracellular acetylation that can be visualized with the reagent of the present invention is not particularly limited, for example, an acetylation that is advanced with acetyl-CoA, such as acetylation of histone with acetyl-CoA, can be a preferred object. For example, the reagent of the present invention can be especially preferably used for visualizing an acetylation in which acetyl-CoA acts as an acetylating agent. As another aspect, acetyl-CoA produced in mitochondria can be measured with the reagent of the present invention. The term "measurement" used in the present specification should be construed in its broadest sense, including determinations, tests, detections, and the like performed for the purpose of quantification, qualification, diagnosis or the like.

In order to visualize an acetylation by using the reagent of the present invention, the rhodamine derivative and the acylation catalyst or acylation reaction-promoting agent may be introduced into a cell, and fluorescence of the rhodamine derivative intracellularly acetylated may be measured. Acetyl-CoA in a cell, preferably in mitochondria, can be similarly measured. A specific example of visualization of acetylation is shown in the section of examples of this specification. Those skilled in the art can easily visualize an intracellular acetylation by performing imaging with choosing appropriate reagents and conditions according to the type of the target acetylation with reference to the specific explanations of the section of examples in this specification.

For example, the reagent of the present invention can be dissolved in an aqueous medium such as physiological saline and buffer, or a mixture of a water-miscible organic solvent such as ethanol, acetone, ethylene glycol, dimethyl sulfoxide, and dimethylformamide, and an aqueous medium, the solution can be added to an appropriate buffer containing cells or tissue, and imaging can be performed on the basis of fluorescence spectra measured before and after the addition. When a reagent containing the rhodamine derivative, and the acylation catalyst or acylation reaction-promoting agent is used as the reagent for visualization, a single solution containing these substances can be prepared and used. Alternatively, the visualization can also be performed by preparing separate solutions of the rhodamine derivative and the acylation catalyst or acylation reaction-promoting agent, and separately contacting those solutions with cells or tissue. In the latter case, the visualization can be performed by simultaneously contacting two kinds of the solutions with cells or tissue, or by contacting two kinds of the solutions with cells or tissue at different times. Fluorescence can be measured by ordinary methods, and a method of measuring fluorescence spectrum *in vitro,* a method of measuring fluorescence spectrum *in vivo* by using a bioimaging technique, and the like are employable. For example, when quantification is performed, it is desirable to create a calibration curve beforehand in a conventional manner.

The reagent of the present invention may be blended with additives usually used for preparation of reagents to be applied to cells or tissues and used as a composition, if needed. For example, as additives for using the reagent in a physiological environment, such additives as dissolving aid, pH modifier, buffering agent, and isotonic agent can be used, and amounts of these additives can be appropriately chosen by those skilled in the art. Such a composition is provided as a composition of an appropriate form, such as powdery mixture, lyophilization product, granule, tablet, and solution.

### Examples

Hereafter, the present invention will be more specifically explained with reference to examples. However, the scope of the present invention is not limited to the following examples.

### Example 1

A fluorescent probe molecule of which fluorescence is increased by acetylation was designed. It is known that acetylaminofluorescein emits stronger fluorescence compared with aminofluorescein by using a photoelectron transfer (PET) type mechanism. However, if fluorescein is used, phenolic hydroxyl groups may also be acetylated, and it is expected that fluorescence intensity shall be reduced thereby. Therefore, the rhodamine structure was chosen as the fluorophore. Rhodamine is known as a fluorophore that emits fluorescence of around 570 nm and is bright also in an aqueous system. Therefore, a rhodamine having an amine on the benzene moiety, RH-NH₂, was designed, and synthesized by the method described below.

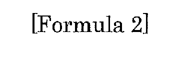

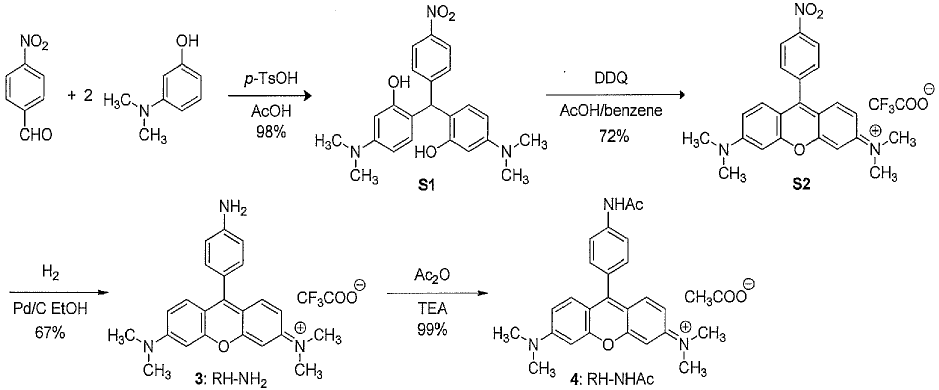

### (a) 6,6'-((4-Nitrophenyl)methylene)bis(3-(dimethylamino)phenol) (S1)

3-(Dimethylamino)phenol (825.8 mg, 6.22 mmol, 2.6 equiv.), p-toluenesulfonic acid (51 mg, 300 µmol, 0.1 equiv.), and acetic acid (20 mL) were added to 4-nitrobenzaldehyde (356.3 mg, 2.36 mmol, 1 equiv.), and the mixture was stirred at 60°C for 14 hours. The reaction mixture was returned to room temperature, and concentrated. Aqueous sodium hydrogencarbonate was added to the residue, and the mixture was extracted with dichloromethane. The organic layers were combined, washed with physiological saline, dried over anhydrous sodium sulfate, and filtered, and the solvent was evaporated. The crude product was purified by silica gel column chromatography (dichloromethane/methanol = 30/1 to 20/1) to obtain the objective substance S1 as orange solid (942 mg, 2.31 mmol, 98%).
¹H-NMR (500 MHz, CD₃OD): δ ppm 2.85 (s, 12H), 6.00 (s, 1H), 6.21 (dd, 2H, J = 8.6 Hz, 2.3 Hz), 6.28 (d, 2H, J = 2.3 Hz), 6.55 (d, 2H, J = 8.6 Hz), 7.23 (d, 2H, J = 9.2 Hz), 8.06 (d, 2H, J = 8.6 Hz)
¹³C-NMR (125 MHz, CD₃OD): δ ppm 41.2, 43.7, 101.7, 105.9, 120.0, 123.8, 131.0, 131.7, 147.1, 152.3, 155.9, 156.5
LR-ESI-MS: 408 (MH⁺)

### (b) N-(6-(Dimethylamino)-9-(4-nitrophenyl)-3H-xanthen-3-ylidene)-N-methylmethanaminium trifluoroacetate (S2)

2,3-Dichloro-5,6-dicyano-p-benzoquinone (DDQ, 247.8 mg, 1.09 mmol, 2 equiv.) was added to S1 (210.8 mg, 0.517 mmol) in benzene/acetic acid (1/1 v/v, 12 mL), and the mixture was stirred at room temperature for 3 hours. The reaction mixture was concentrated, and the residue was subjected to silica gel column chromatography (dichloromethane/methanol/trifluoroacetic acid = 10/1/0.01) to obtain a crude product (197.6 mg). The crude product was purified by reverse phase column chromatography (methanol) to obtain the objective substance S2 as purple solid (187.3 mg, 0.374 mmol, 72%).
¹H-NMR (CD₃OD, 500 MHz): δ ppm 3.23 (s, 12H), 7.00 (d, 2H, J = 2.3 Hz), 7.11 (dd, 2H, J = 9.2 Hz, 2.3 Hz), 7.30 (d, 2H, J = 9.7 Hz), 7.77 (d, 2H, J = 8.6 Hz), 8.53 (d, 2H, J = 8.6 Hz)
¹³C-NMR (125 MHz, CD₃OD): δ ppm 41.1, 97.8, 102.2, 114.3, 114.6, 115.9, 125.0, 130.2, 132.2, 132.3, 140.3, 150.4, 152.8, 159.0
LR-ESI-MS: 388 [M-CF₃COO]⁺
HR-ESI-MS: calced for C₂₃H₂₂N₃O₃⁺ [M-CF₃COO]⁺ 388.1656, found 388.1674

### (c) N-(9-(4-Aminophenyl)-6-(dimethylamino)-3H-xanthen-3-ylidene)-N-methylmethanaminium trifluoroacetate (3)

S2 (80.1 mg, 0.160 mmol) was dissolved in ethanol (10 mL) under an argon atmosphere, 10% Pd/C (43.1 mg) was added to the solution, and hydrogen gas was introduced into the mixture. The reaction mixture was stirred at room temperature for 2 hours, and then filtered through Celite, and the filtrate was concentrated to obtain a crude product. The crude product was purified by reverse phase column chromatography (60% methanol to 100% methanol) to obtain the objective compound 3 as purple solid (50.5 mg, 0.107 mmol, 67%).
¹H-NMR (500 MHz, CD₃OD): δ ppm 3.34 (s, 12H), 6.85-6.92 (m, 4H), 7.06 (dd, 2H, J = 9.6 Hz, 2.8 Hz), 7.26 (d, 2H, J = 8.2 Hz), 7.63 (d, 2H, J = 9.6 Hz)
¹³C-NMR (125 MHz, CD₃OD): δ ppm 40.8, 97.5, 114.4, 115.0, 115.2, 120.9, 125.0, 132.2, 132.3, 133.2, 133.4, 152.7, 158.6, 161.1
LR-ESI-MS 358 [M-CF₃COO]⁺
HR-ESI-MS: calced for C₂₃H₂₄N₃O⁺ [M-CF₃COO]⁺ 358.1914, found 358.1915

### (d) N-(9-(4-Acetamidophenyl)-6-(dimethylamino)-3H-xanthen-3-ylidene)-N-methylmethanaminium acetate (4)

Acetic anhydride (0.5 mL) and triethylamine (TEA, 50 µL) were added to the compound 3 (2.7 mg, 5.72 µmol), and the mixture was stirred at room temperature for 5 hours. The reaction mixture was concentrated under reduced pressure, and the residue was purified by reverse phase column chromatography (methanol) to obtain the objective compound 4 as purple solid (2.6 mg, 5.66 µmol, 99%).
¹H-NMR (CD₃OD, 500 MHz): δ ppm 2.14 (s, 3H), 2.27 (s, 3H), 3.24 (s, 12H), 6.85-6.70 (m, 2H), 7.00-7.05 (m, 2H), 7.38-7.42 (m, 4H), 7.79-7.82 (m, 2H)
¹³C-NMR (CD₃OD, 125 MHz): δ ppm 20.0, 24.0, 40.9, 97.6, 114.2, 114.6, 115.5, 115.9, 120.9, 131.8, 132.3, 132.9, 158.9, 159.3, 169.8, 172.1
LR-ESI-MS: 400 [M-CH₃COO]⁺
HR-ESI-MS: calced for C₂₅H₂₆N₃O₂⁺ [M-CH₃COO]⁺ 400.2020, found 400.2028

The compound 3 (RH-NH₂) obtained had the absorption maximum (ε = 71400 M⁻¹cm⁻¹) at 546 nm in PBS, fluorescence maximum at 570 nm with excitation at 550 nm, and fluorescence quantum yield of 4.9 x 10⁻⁴ as measured in PBS using rhodamine B as a control. The acetylated compound 4 (RH-NHAc) had the absorption maximum (ε = 35500 M⁻¹cm⁻¹) at 553 nm, fluorescence maximum at 572 nm with excitation at 550 nm, and fluorescence quantum yield of 0.12.

### Example 2

The compound 3 (1 µM) and an acylation catalyst or acylation reaction-promoting agent (10 mM) were dissolved in PBS (pH 7.4, containing 1% DMSO), N-methoxydiacetamide (NMD, 0.1M) was added as an acetylating agent 1 minute afterward, and change of fluorescence was measured over time at 25°C. When dimethylaminopyridine (DMAP) or tributylphosphine (PBu3) was added as the acylation catalyst or acylation reaction-promoting agent, larger increase in fluorescence intensity was observed over time compared with that observed without addition of the acylation catalyst or acylation reaction-promoting agent. Tributylphosphine gave a fluorescence intensity about 5 times higher than that observed without the addition at 5 minutes after the addition, and this result means that the addition of tributylphosphine achieved about 5.5 times of increase in the reaction rate compared with the reaction rate observed at the start of the reaction without the addition of the catalyst and acylation reaction-promoting agent. Thus, it was demonstrated that tributylphosphine acts as an effective acylation catalyst or acylation reaction-promoting agent for the compound 3 in an aqueous system.

### Example 3

3-(Acetylthio)propane-1-sulfonic acid sodium salt (6) was synthesized as an analogue compound of acetyl-CoA by the following method. It is considered that this compound functions as an acetylating agent like acetyl-CoA through exchange of thioester.

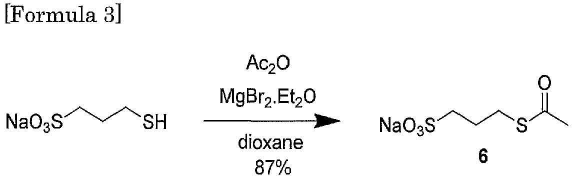

MgBr₂-ether complex (131 mg, 0.50 mmol, 0.07 eq.), acetic anhydride (6 mL), and dioxane (6 mL) were added to 3-mercapto-1-propanesulfonic acid sodium salt (1.38 g, 7.72 mmol), and the obtained mixture was stirred for 17 hours. Methanol and water were successively added to terminate the reaction, the reaction mixture was concentrated under reduced pressure, then the obtained solid was suspended in methanol, and the precipitates were remove by filtration. The filtrate was concentrated to obtain the compound 6 as substantially pure white solid (1.63 g, 7.41 mmol, 96 %).
¹H-NMR (500 MHz, D₂O): δ ppm 1.90-1.95 (m, 2H), 1.99 (s, 3H), 2.56 (t, 3H, *J =* 6.9 Hz), 2.92 (t, 3H, *J =* 8.0 Hz)
¹³C-NMR (125 MHz, D₂O) δ ppm 21.1, 23.2, 29.0, 50.1, 177.3

The compound 3 (1 µM) was mixed with tributylphosphine (10 mM) as the acylation catalyst or acylation reaction-promoting agent, the compound 6 was added 1 minute afterward, and advance of the acetylation was measured over time at 25°C in PBS (pH 7.4, 1% DMSO) in the same manner as that of Example 2. Fluorescence of 575 nm was measured with excitation at 550 nm. Rapid increase of fluorescence was obtained by the effect of the acylation catalyst or acylation reaction-promoting agent (Fig. 1). Correlation was observed between the fluorescence intensity and the concentration of the acetylating agent 1 minute after the addition of the acetylating agent (Fig. 2). These results demonstrated that, by using this reaction system, the acetylating agent at a millimole level can be detected at the time point after 1 minute of reaction time, and it was considered that the acetylation can be observed under biological conditions with the combination of the compound 3 and tributylphosphine.

### Example 4

In order to visualize an intracellular acetylation, the following experiment was conducted by using the compound 3 as the rhodamine derivative. It is known that a positively charged rhodamine derivative structure such as tetramethylrhodamine migrates into cells and localizes in mitochondria (J. Circ. Res., 95, pp.239-252, 2004; J. Biol. Chem., 264, pp.8171-8178, 1989; Plos. One., 6, e23684, 2011; as especially for localization of 5-aminorhodamine derivatives in mitochondria, see, GB2283744 and US5686261). The compound 3 (10 µM) was allowed to act on the HeLa cells at 37°C for 30 minutes in the Hanks' balanced salt solution (HBSS, 137 mM NaCl, 5.4 mM KCl, 1.3 mM CaCl₂, 0.5 mM MgCl₂, 0.4 mM MgSO₄, 0.3 mM Na₂HPO₄, 0.4 mM KH₂PO₄, 4.2 mM NaHCO₃, 5.6 mM D-glucose, 5 mM HEPES, adjusted to pH 7.4 with NaOH), the cells were washed twice with HBSS, and then fluorescence was measured. As a result, uniform staining image of the inside of the cells, indicating localization of the compound in the mitochondria, was obtained.

The localization in the mitochondria of the HeLa cells was visualized by expression of TagCFP-Mito, which is a cyan fluorescent protein, and thereby confirmed. The HeLa cells were transfected with the pTagCFP-Mito vector (Evrogen) using Lipofectamine LTX (Invitrogen), and fluorescence was measured from 24 hours thereafter. The compound 3 was excited at 559 nm, and fluorescence of 580 to 680 nm was measured. TagCFP-Mito was excited at 440 nm, and fluorescence of 460 to 500 nm was measured. The fluorescence images were analyzed by using FluoView (Olympus), and fluorescence intensity was calculated as average intensity in a predetermined region of interest (ROI) containing the cell body of each cell. The results are shown in Fig. 3. The fluorescence image of the compound 3 was found to be well consistent with the fluorescence image of the cyan fluorescence protein expressed in the mitochondria.

Even when the compound 3 was added to the HeLa cells at 37°C, enhancement of fluorescence intensity was not observed over time in the absence of the acylation catalyst or acylation reaction-promoting agent. Whilst, when tributylphosphine (5 mM) was added to the medium, enhancement of intracellular fluorescence intensity was observed (Figs. 4, (a), (c), and (d)). In view of the results of the model acetylation shown in Example 3, it is considered that enhancement of the intracellular fluorescence intensity was originated in acetylation advanced by physiological acetyl-CoA in the inside of mitochondria. When N-methoxydiacetamide (10 mM) was added to the cells as an acetylating agent at the time point of 7 minutes, the fluorescence intensity was further enhanced (Figs. 4, (a) and (e)). Therefore, advance of the intracellular acetylation was successfully visualized by the compound 3 together with the physiological acetyl-CoA and the additionally added acetylating agent. It is known that the acetyl-CoA concentration in mitochondria is higher than the average intracellular acetyl-CoA concentration (J. Gen., Microbiol., 134, pp.2249-2253, 1988), and the aforementioned results are consistent with this finding.

### Industrial Applicability

By imaging acetyl-CoA using the reagent of the present invention, and confirming distribution and localization thereof, it will be possible, for example, to pathologically and physiologically elucidate a cause of onset or advance of pathological conditions of diseases such as arteriosclerosis and hyperlipidemia at a cellular level.

## Claims

1. A reagent for visualizing an intracellular acetylation, which comprises a combination of a rhodamine derivative that is substantially non-fluorescent before being acetylated, and emits strong fluorescence after being acetylated, and an acylation catalyst and/or an acylation reaction-promoting agent.

2. The reagent according to claim 1, wherein the intracellular acetylation is an acetylation advanced by acetyl-CoA.

3. A reagent for measuring intracellular acetyl-CoA, which comprises a combination of a rhodamine derivative that is substantially non-fluorescent before being acetylated, and emits strong fluorescence after being acetylated, and an acylation catalyst and/or an acylation reaction-promoting agent.

4. The reagent according to any one of claims 1 to 3, wherein the rhodamine derivative is a rhodamine derivative represented by the following general formula (I): 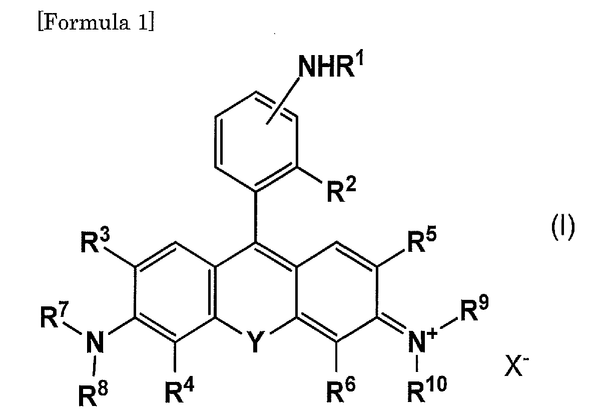 wherein, in the formula, R¹ represents hydrogen atom or a C₁₋₆ alkyl group; R² represents hydrogen atom, a C₁₋₆ alkyl group, or carboxyl group; R³ and R⁵ independently represent hydrogen atom, a halogen atom, or a C₁₋₆ alkyl group; R⁴ and R⁶ independently represent hydrogen atom or a C₁₋₆ alkyl group; R⁷ and R⁸ independently represent a C₁₋₆ alkyl group, but the alkyl group represented by R⁷ and the alkyl group represented by R³ may bind together to form a 5- to 7-membered ring, and/or the alkyl group represented by R⁸ and the alkyl group represented by R⁴ may bind together to form a 5- to 7-membered ring; R⁹ and R¹⁰ independently represent a C₁₋₆ alkyl group, but the alkyl group represented by R⁹ and the alkyl group represented by R⁵ may bind together to form a 5- to 7-membered ring, and/or the alkyl group represented by R¹⁰ and the alkyl group represented by R⁶ may bind together to form a 5- to 7-membered ring; X⁻ represents a counter ion; and Y represents oxygen atom or M(R¹¹)(R¹²) (M represents silicon atom, germanium atom, or tin atom; and R¹¹ and R¹² independently represents a C₁₋₆ alkyl group).

5. The reagent according to claim 4, wherein R¹ is hydrogen atom or a C₁₋₆ alkyl group; R² is hydrogen atom, a C₁₋₆ alkyl group, or carboxyl group; R³ and R⁵ are hydrogen atoms; R⁴ and R⁶ are hydrogen atoms; R⁷ and R⁸ are independently C₁₋₆ alkyl groups; R⁹ and R¹⁰ are independently C₁₋₆ alkyl groups; X⁻ is a counter ion; and Y is oxygen atom.

6. The reagent according to claim 4, wherein R¹ is hydrogen atom; R² is hydrogen atom; R³, R⁴, R⁵, and R⁶ are hydrogen atoms; R⁷, R⁸, R⁹, and R¹⁰ are methyl groups; X⁻ is a counter ion; and Y is oxygen atom.

7. The reagent according to any one of claims 1 to 6, wherein the acylation catalyst or acylation reaction-promoting agent is an acylation catalyst or acylation reaction-promoting agent selected from the group consisting of dialkylaminopyridines, cyclic tertiary organic amines, and phosphines.

8. The reagent according to claim 6, wherein the acylation catalyst or acylation reaction-promoting agent is dimethylaminopyridine, 1,4-diazabicyclo[2.2.2]octane, or tributylphosphine.

9. A method for visualizing an intracellular acetylation, which comprises the step of introducing a combination of a rhodamine derivative that is substantially non-fluorescent before being acetylated, and emits strong fluorescence after being acetylated, and an acylation catalyst and/or an acylation reaction-promoting agent into a cell, and the step of measuring fluorescence of the rhodamine derivative having been acetylated.
